# EUROPEAN PATENT APPLICATION

(11) **EP 2 302 353 A1**
(43) Date of publication of application: **30.03.2011**
(21) Application number: 09290744.3
(22) Date of filing: 28.09.2009
(51) Int. Cl.: G01N 13/00, G01N 33/15, C12M 3/06

(54) **Online TEER measurement in a system for permeation determination by means of a flow-through permeation cell (FTPC) having structurally integrated electrodes**

(71) Applicant: Sanofi-Aventis, 75013 Paris (FR)
(72) Inventor: Walk, Jutta, 65926 Frankfurt am Main (DE); Eichinger, Thomas, 65926 Frankfurt am Main (DE); Balbach, Stefan, 65926 Frankfurt am Main (DE); Loos, Petra, 65926 Frankfurt am main (DE); Lehr, Claus-Michael, 66125 Saarbrücken (DE); Schaefer, Ulrich, 66280 Sulzbach (DE); Muendoerfer, Marco, 66111 Saarbrücken (DE)
(74) Representative: Sieber, Frank

(57) **Abstract**

The instant invention concerns a novel method for measuring the permeability and integrity of a cellular layer with respect to a sample of interest, novel devices for the determination of the transepithelial electrical resistance (TEER), for the measurement of permeation and the combination of dissolution (release) and permeation and their use in permeation and combined dissolution (release)/permeation measurement systems. The invention teaches a novel flow-through permeation cell (FTPC) having structurally integrated electrodes for the improved design of permeation studies of cellular layers under physiological conditions.

## Description

The instant invention concerns a novel method for measuring the permeability and integrity of a cellular layer with respect to a sample of interest, novel devices for the determination of the transepithelial electrical resistance (TEER), for the measurement of permeation and the combination of dissolution (release) and permeation and their use in permeation and combined dissolution/permeation measurement systems. The invention teaches a novel flow-through permeation cell (FTPC) having structurally integrated electrodes for the improved assay design of permeation studies of cellular layers under physiological conditions.

Many methods and technologies are known for the measurement of permeation on cellular layers under physiological conditions (Ginski & Polli (1999) AAPS PharmSci 1: E3; Kobayashi et al. (2001) lnt J Pharm 221: 87-94; Kataoka et al. (2003) Pharm Res 20: 1674-1680). Furthermore, test methods for dissolution (release) or permeation are well defined in the compendial art, e.g., by USP (United States Pharmacopeia) standards.

Motz et al. Eur J Pharm and Biopharm (2007) 66(2): 286-295 disclose an apparatus for the combined determination of dissolution (release) and permeation, which allows for the combined examination of the in-vitro release of an active ingredient(s) from a drug formulation (or the drug substance itself) and the permeation through a Caco-2 monolayer, two steps that are commonly assessed separately. By means of such a system it is possible to assess the influence of excipients, formulation auxiliaries, active ingredients, or formulation types on release and permeation in one step by the combined use of a compendial flow-through cell (e.g. USP apparatus 4) and a flow-through permeation cell (FTPC).

Caco-2 cells grown on permeable supports are a well known model for drug transport studies and feature biological barrier properties that are close to the human small intestinal epithelium.

Furthermore, Motz et al. Analytica Chimica Acta (2007) 581(1): 174-180 disclose a modified apparatus for the determination of dissolution and permeation properties having an additional module for automation of sampling and detection (SIA module).

The methods of Motz et al. use the determination of TEER, which is a well established, nondestructive and non-invasive in-vitro method for monitoring the integrity of cellular layers, e.g. to monitor progression of growth and maturation of cellular layers that are seeded on permeable supports. Confluence of the cells is pointed out by an increase in the TEER value, and the magnitude of the TEER value correlates with the tightness and impermeability of the cellular layer. In permeation assay methods, TEER measurement is used before and after the permeation experiment in order to control the status and integrity of the cellular layer. The integrity of the cellular layer is a crucial point for every permeation experiment, as the resulting data will only be trustworthy, if the barrier properties of the cellular layer are essentially intact.

A standard method to check the tightness of a cellular layer is the manual measurement of the TEER value before and after a permeation experiment, e.g., inside a multi-well (6, 12, 24, 48, or 96 wells) culture plate (e. g., Transwell®, Corning Inc., New York, USA).

TEER measurement may be performed with an 'Epithelial Voltohmmeter' (EVOM, World Precision Instruments (WPI), Sarasota, Florida, USA) and corresponding so-called chopstick electrodes (e.g., STX-2 electrodes from WPI). Chopstick electrodes feature specially designed shanks so that after insertion into the apical and the basolateral compartment of a cellular layer carrier (e.g., Transwell®) that is located inside the appropriate multi-well plate, a direct contact between the apical electrode and the cellular layer is prevented. Further, automated PC-controlled TEER measurement may be performed by WPI's REMS system providing a user-specified software for the control of the measurement.

Usually, the first measurement before the start of a permeation experiment is performed after the replacement of the growth medium by a physiologic buffer solution and succession of a certain equilibration time. The TEER is measured in the respective well of the multi-well plate to confirm confluence and integrity of the cellular layer at the beg.inning. Then, the cellular layer carrier is taken out of the plate and inserted into the FTPC in order to start with the permeation experiment. As soon as the experiment is finished, the cellular layer carrier is removed from the FTPC and placed in a new well of a plate for TEER measuring, again in physiological buffer. Now, the second value of TEER is determined to control the status and the integrity of the cellular layer at the end of the permeation experiment.

Wegener et al. (2004) Biotechniques 37(4):590-7 disclosed a system for the automated monitoring of cellular layers by measuring TEER (CellZscope®, Nanoanalytics, Münster, Germany). This system provides information on the barrier properties and the differentiation stage of cell layers by measuring TEER. It is suited for studying the influence of substances such as drugs, toxins etc. on the barrier function of cellular layers, e.g., throughout the period of cell growth.

However, none of the available systems allows the continuous monitoring of TEER in a dynamic permeation or dissolution (release)/permeation system which requires the integration of a FTPC in an open or closed system, yet allowing to take samples. In contrast to a sheer permeation system a combined dissolution (release)/permeation system allows the assessment of various types of samples of interest such as formulations or drug substances (active ingredients) and is not restricted to liquids only.

Accordingly, the instant invention addresses the problem that during the course of the examination of samples of interest such as drugs (e.g., pharmaceutically active ingredients) or drug products (e.g., pharmaceutical formulations and/or excipients) by known permeation or dissolution/permeation methods, the integrity of the incorporated cellular layers needs to be controlled throughout the course of the experiment in order to allow correct interpretation of the resulting data.

By use of the currently available systems for the measurement of permeation through a cellular layer, the integrity of the cellular layer needs to be determined before and after the performance of the permeation experiment. This approach, however, delivers insufficient information with respect to potential damage or other adverse effects on the cellular layer during the course of the experiment. Any violation of the cellular layer or any adverse effect on the cellular layer during the course of the permeation experiment will only be noticed after the termination of the experiment. A potential short-term, reversible vitiation of the cells that might-trigger a temporary permeation enhancement would retain unnoticed.

Such violation of the cellular layer might result during the assay, due to many reasons, e.g. due to mechanical damage during insertion into or removal of the carrier from the FTPC, e.g., by inadvertent contact of cellular layer and electrodes, due to other mechanical constraints, or due to changes in the experimental conditions (increase in concentration of any component of the sample of interest, pH value, osmolarity, or any other buffer parameter, pressure, etc) during the permeation experiment. Furthermore, as the measurement of TEER before and after an experiment takes place outside of the FTPC which means that the cellular layer carrier (e.g., a Transwell® plate) has to be removed from the FTPC and inserted again into the FTPC. Thereby, the buffer solution has to be replenished, which further poses stress to the cellular layer, and it may be critical to conclude that the TEER before and after relocation of the cellular layer is the same.

Therefore, it appears that two TEER values, one before and one after the permeation experiment providing information about the status of the cellular layer is not really satisfying to monitor the cellular integrity during the whole course of a permeation experiment.

Accordingly, there has been an urgent need for providing a tool that allows monitoring of the current status of the cellular layer during the progression of a permeation experiment. It was therefore the task of the instant invention to provide an in-vitro assay method which allows the continuous online determination of the status and integrity of a cellular layer during the whole course of a permeation experiment.

By use of instant invention, any alteration or damage in the cellular layer can be detected as soon as it occurs, when such alteration or damage is caused, thereby providing valuable additional information.

The duration of an experiment may be adjusted according to the quality and status of the incorporated cellular layer as it can now be monitored in greater detail how long a cellular layer may maintain its integrity during the course of a permeation experiment.

The methods according to instant invention allow the simultaneous investigation of the release or dissolution of a sample of interest, e.g., an active pharmaceutical ingredient (API) from a formulation (or the release or dissolution from the drug substance itself) and its permeation through a biological barrier, e,g., a cellular layer or a membrane, or an artificial membrane such as a-filter membrane coated with various lipoidal compounds or a PAMPA-like system (Parallel Artificial Membrane Permeation Assay), thereby monitoring online the status and integrity of the cellular layer by TEER. Moreover, qualitative and quantitative information may be gathered regarding the influence of drugs (i.e., pharmaceutically active ingredients), excipients (e.g., pharmaceutical formulation auxiliaries), and/or experimental conditions on the biological barrier properties of the cellular layer and the resulting status. Thus, dissolution (release) and/or permeation kinetics of drugs or excipients can be analyzed in greater detail.

Online TEER measurement inside the apparatus is a suitable method for in-line-control of the barrier integrity throughout each experiment and can be used as a tool to analyse impacts of excipients that may modulate barrier properties such as intestinal permeability.

Furthermore, the instant invention allows software controlled continuous online measurement of TEER during the whole course of the permeation experiment in arbitrary chosen time intervals. Further automation by a suitable control unit may reduce manpower during the permeation assay performance and improve the quality of the obtained results.

Accordingly, the instant invention will lead to a significant improvement of the quality and the interpretability of the results of permeation or dissolution/permeation experiments, thereby providing an improved tool in drug and formulation selection in pharmaceutical drug development.

The above mentioned shortcomings have been overcome according to the invention by the subject matter as defined in the claims. Accordingly, the claimed invention provides in a first embodiment a method for measuring the permeability of a cellular layer for a sample of interest comprising the steps of
a) providing a flow-through permeation cell (FTPC) comprising suitable electrodes;
b) introducing a suitable carrier system for said cellular layer into said FTPC;
c) filling the system with a suitable buffer solution at the apical and the basolateral side of said cellular layer;
d) introducing said sample of interest at the apical side of said cellular layer;
e) taking samples of liquid at the basolateral side or at the apical and the basolateral side of the cellular layer in certain time intervals;
f) determining the amount of said sample of interest in said samples of liquid taken and
g) determining the transepithelial electrical resistance (TEER) of the cellular layer in certain time intervals during the course of the permeation measurement,
whereby the said electrodes are structurally integrated into the said FTPC.

Optionally, if the sample of interest is a solid, the method according to the invention may further comprise the preceding step of providing a dissolution chamber, e.g., USP apparatus 4, wherein the said sample of interest is placed. USP Apparatus 4 is a flow-through cell containing the sample and is fed with dissolution buffer from a buffer reservoir. Hereby, at least part of the outlet of the dissolution chamber serves as the inlet of the FTPC for the sample of interest. This additional step in the method according to the invention would additionally allow the determination of dissolution properties of the sample of interest by taking samples of liquid at the outlet of the dissolution chamber in certain time intervals during the course of the permeation measurement and determining the amount of the sample of interest therein.

In another embodiment of the invention one or more assay parameter(s), e.g., buffer flow rates between the assay modules, concentration of the sample of interest, buffer composition, temperature, and/or pressure, are controlled by a control unit, which is connected to the system components, and which may be connected via one or more A/D converters to the components of the assay system and to a personal computer. Thereby, a suitable software may allow the user to enter predefined values of all required assay parameters and to perform the assay in accordance with a predefined assay profile.
The term control unit" according to the instant invention may include any microprocessor unit within or outside a personal computer which is able to communicate with the hardware components of the assay system via suitable interfaces or A/D converters and allows interaction with the user via a monitor and a keyboard input or the like. By means of a suitable software the user may define the assay parameters (e.g., flow control via suitable pump systems, control of test parameters like temperature, pressure, buffer composition, etc.) and assay preparation and performance. Further tasks controlled by the control unit may include instrument maintenance, test calibrations, quality control displays and evaluations, adjustment of assay parameters.

In yet another embodiment of instant invention data acquisition and/or processing of obtained data is supported by the control unit in order to further optimize automation and time management of the assay performance.

The term "automation step" according to instant invention may include, but is not limited to, e.g., time points and time intervals of TEER measurement, sample taking at the apical (A) and/or basolateral (B) compartment of the FTPC or at or after the dissolution module (2) at the dissolution sampling port (D). Further parameters concerning sample analysis or sample detection, and/or data processing may also be subject to control by the control unit and may be covered by the term "automation step". Furthermore, the software used may allow the user to enter predefined values of assay analysis parameters and to perform the data processing for the assay analysis in accordance with a predefined assay analysis profile.

The term "transepithelial electrical resistance" ('TEER") according to the instant invention may not be understood to be only limited to the transepithelial electrical resistance, but shall include any electrical indicator, which is suitable to identify directly or indirectly the transcellular and/or paracellular electrical property of the cellular layer under investigation, preferably the transcellular and/or paracellular electrical resistance and/or the transcellular and/or paracellular electrical capacitance or impendance.

The term "suitable etectrode" according to the instant invention may include any electrode which is suitable to perform the measurement of TEER inside a FTPC and is structurally integrated in the FTPC. The electrode may be made of iron, cooper, silver, gold, and/or platinum or any other suitable material. If an electrode is made of iron, stainless steel is preferred. The electrodes shall be placed at positions in the apical and basolateral compartment of the cellular carrier system to allow the detection of a signal, whereby it is favorable to design the electrical field as homogenous as possible. If a mufti-well cellular layer carrier system is used, the basolateral electrode may be designed as one common electrode for all wells on the cellular layer carrier, whereas each single of the apical electrodes is separately controlled by the control unit.

The term "structurally integrated" according to the instant invention preferably means that the electrode is partly or fully, optionally reversibly, integrated into the body of the FTPC, in order to avoid the need for manipulation during the measurement. Optionally, part of the electrode may be integrated in the cellular layer carrier. It may be favorable to allow removal or exchange of the electrodes from the FTPC and/or cellular layer carrier. The body of the FTPC is preferably made of a suitable dielectric, preferably a plastic material, e.g., polyetheretherketone (PEEK). In another preferred embodiment at least the basolateral compartment of the FTPC allows housing of a magnetic stirrer.

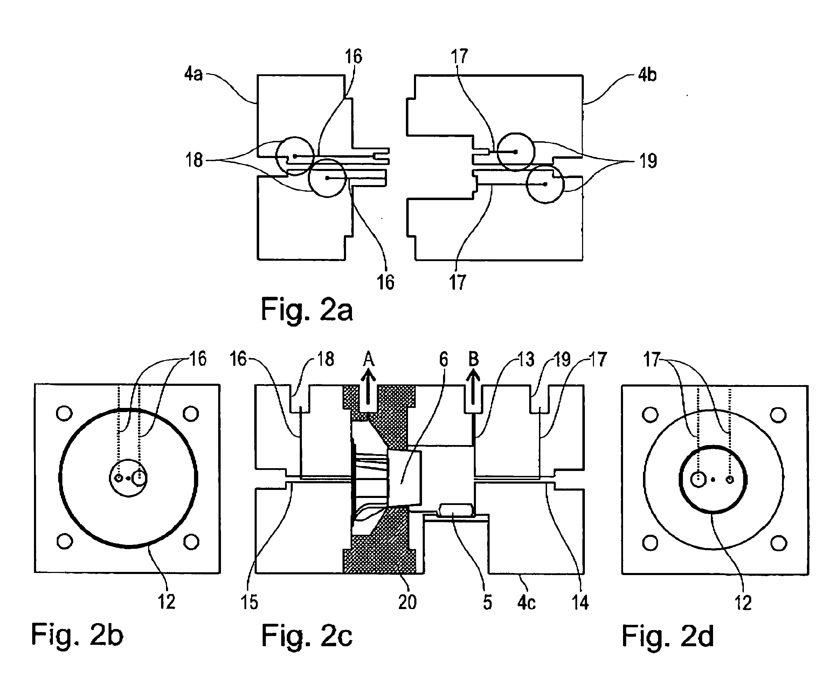

The term "carrier system for a cellular layer" or "cellular layer carrier" according to the instant invention may include any cellular layer carrier which can be integrated into a FTPC for the performance of TEER measurement. Known carrier systems have a permeable membrane exhibiting pore diameters of about 0.1 to 10 µm and providing multi-well plates of 6. 12, and 24, or more wells, each well having an apical and basolateral compartment separated by a permeable membrane. Such cellular layer carriers are commercially available, e.g., from Corning Costar, Greiner, Millipore, BD Biosciences, Nunc and others. Alternatively, cellular carrier systems may be used, which have already complete or partly integrated electrodes which fit to the adapters in the FTPC in order to avoid the risk of damaging the cellular layer while assembly/disassembly the measurement equipment. Such carrier systems are known, e.g., from DE 102007003585 A1, including multi-well carrier systems. If a multi-well cellular layer carrier shall be integrated into the FTPC according to instant invention, a modified electrode geometry would be required compared to the one shown in Figure 2 as well as a control unit and modified sampling geometry which allows individually addressable wells for obtaining the complete data set of information.

The term "cellular layer according to the instant invention may include any biological barrier forming cell or tissue, preferably a mono-, bi-, tri-, or multilayer, preferably a monolayer of one or more cell types or cell aggregates, more preferred of endothelial, such as mesothelial or epithelial cells, e.g., of gastric, renal, brain, vascular, or alveolar origin. Usually, a confluent monolayer is favorable. Suitable cell types are, for example, Caco-2, Calu-3, A549, MDCK, beside many others. Further, the term "cellular layer" according to the invention may include any biological or artificial barrier formed by biological or artificial membranes, e.g., lipid double-layers. Usually, the cellular layer is grown or placed on a carrier, optionally, by means of a suitable paper filter or plastic carrier.

It is one embodiment of instant invention to have a FTPC which is temperature controllable, in order to maintain the temperature at a predefined value during the performance of the measurement. This may be obtained, e.g., by a circuit of thermostatic fluid within the FTPC or by a temperature controllable chamber surrounding the FTCP. Usually, the temperature shall be maintained within a physiological acceptable range of about 0°C to 40°C, preferably of 25°C to 38°C, depending on the cell type under investigation.

In yet another embodiment of instant invention, the measurement of the TEER is performed in pre-defined time intervals.

The term "time interval" according to the instant invention may include time intervals of about 1 to 60 sec. or, alternatively, 1 to 60 min. Accordingly, the whole duration of a permeation experiment may be in the order of magnitude from minutes to days. The time interval chosen shall be in accordance with the timely requirements of the system under investigation, and depends on the stability of the cellular layer, the duration of the dissolution and/or permeation processes of interest.

Another object of instant invention is a device for measuring the transepithelial electrical resistance (TEER), **characterized in that** it comprises
a) a flow-through permeation cell (FTPC) designed to allow the introduction of a carrier system for a cellular layer;
b) at least one pair of electrodes, which are in contact with the apical and basolateral compartments of the said cellular layer whereby
c) the said electrodes are at least partly structurally integrated into the said FTPC.

Optionally, the device for measuring the TEER according to the invention comprises a liquid outlet at the at least basolateral side of the cellular layer for sample taking. Accordingly, another liquid outlet may be placed at the apical side of the cellular layer in order to allow sample taking.

In another embodiment the device for measuring the TEER according to the invention is under control of a control unit for the adjustment of assay parameters, device maintenance, test calibrations, data acquisition and/or processing of obtained data and may include, but are not limited to, e.g., sample taking at the apical (A) and/or basolateral (B) compartment of the FTPC, control of test parameters like temperature, pressure, buffer composition, etc..

Another object of instant invention is a device for the measurement of permeation of a sample of interest through a cellular layer comprising the device for measuring the TEER according to the invention having a liquid outlet at the at least basolateral side of the cellular layer for sample taking; which is optionally under control of a control unit for the adjustment of assay parameters, device maintenance, test calibrations, data acquisition and/or processing of the obtained data.

Still another object of instant invention is a device for the combined measurement of dissolution of a sample of interest and the measurement of its permeation through a cellular layer comprising device for measuring the TEER according to the invention having a liquid outlet at the at least basolateral side of the cellular layer for sample taking; which is optionally under control of a control unit for the adjustment of assay parameters, device maintenance, test calibrations, data acquisition and/or processing of the obtained data, further having a preceding dissolution chamber, wherein the said sample of interest is placed for the determination of its dissolution properties and having an opening which allows to take samples of liquid at the outlet of the dissolution chamber at certain time intervals during the course of the permeation measurement.

A further object of instant invention is the use of electrodes for the at least partly structural integration into a flow-through permeation cell (FTPC) designed to comprise a carrier system for a cellular layer for the assessment of the transepithelial electrical resistance.

And yet another object of instant invention is the use of one of the devices according to the invention for the assessment of diffusion and/or permeation properties of a sample of interest through a cellular layer.

### Description of the Figures

Figure 1 shows a schematic overview of the device according to instant invention for the combined measurement of dissolution and permeation. A dissolution module (2) comprising a flow through dissolution cell (Apparatus 4 USP) (1) is linked with a permeation module (3) comprising the FTPC (4) according to the invention connected via a stream splitter (7).
Figure 2 shows the implementations of the apical and basolateral bricks in the FTPC for the introduction of electrodes (16, 17) and electrode wiring
Fig. 2 (a) shows a top view on the apical and basolateral components of the FTPC (4) and the integrated electrodes (16, 17) therein. The circles indicate the space that is needed for mounting 1/4'-28G flanges fitting adapters (18, 19). The cellular layer carrier (6) is not shown in this view.
Fig. 2 (b) shows an interior view of the integrated apical electrodes (16) and seal ring (12)
Fig. 2 (c) shows a side view of the FTPC (4) and the cellular layer carrier holder (20) with a mounted cellular layer carrier (6). A seal is located between carrier (6) and carrier holder (20) (not shown).
Fig. 2 (d) shows an interior view of the integrated basolateral electrodes (17) and seal ring (12)

### Reference Signs

- 1: apparatus 4 USP
- 2: dissolution module
- 3: permeation module
- 4: FTPC flow-through permeation cell
- 4a: apical part of the FTPC
- 4b: basolateral part of the FTPC
- 4c: FTPC with the cellular layer carrier
- 5: magnetic stirrer
- 6: cellular layer carrier
- 7: stream splitter
- 8: buffer reservoir
- 9: buffer solution
- 10: sample
- 12: seal
- 13: sampling outlet B
- 14: buffer reservoir inlet
- 15: fluid inlet
- 16: apical electrodes
- 17: basolateral electrodes
- 18: apical electrode adapter
- 19: basolateral electrode adapter
- 20: cellular layer carrier holder
- A: apical sampling port
- B: basolateral sampling port
- D: dissolution sampling port
- Arrows: indicate the direction of flow.

### Examples

### Device Setup

In contrast to the apparatus and setup that has been described by Motz et al. Eur J Pharm and Biopharm (2007) 66(2): 286-295 which disclosure content is herewith expressly referred to and is incorporated by reference, the following modifications have been introduced.

The device design was basted on an Epithelial Voltohmmeter (EVOM, World Precision Instruments, WPI, Sarasota, Florida, USA). In particular, an EVOMX of WPI was used which features an additional BNC output for data acquisition. The electrodes (16, 17) were structurally integrated inside the FTPC (4) to enable the measurement of the TEER values during the course of the experiment (see Fig 2). The EVOMX used had a four point probe setup for voltage measurement. As voltage sensors sintered silver-silver chloride bare sensor electrodes (In Vivo Metric, Healdsburg, California, USA) and as current electrodes pure silver wire have been used and were integrated in the apical and the basolateral brick of the FTPC as shown in Fig. 2.

Drillings were added to the apical and the basolateral brick of the FTPC that allowed for the necessary wiring and the seamless implementation of the electrodes (16, 17) into the inner surface of the units. At the outside of the bricks screw ports with ¼'-28G threads for connection with flangeless fitting adapters (18, 19) have been integrated (see Fig 2). The adapters (18, 19) served as spacers for plugging in the cables to the EVOMX and provided a proper sealing against intrusion of water from the surrounding water bath. In the heads of these adapters (18, 19) plugs with M5 screw threads that are connected to the internal wiring have been mounted. Inside the adapters (18, 19) the wiring was led through a piece of 1/16" polymeric tubing which was tightened by means of a flangeless ferrule at the moment that the adapter was screwed in. To prevent a leakage line between wire and polymeric tubing, the wire has been sealed inside the tubing by means of a drop of glue. At the basolateral brick the electrodes have been integrated into the backplane of the interior cavity. With respect to the apical brick it turned out that it was better to mount the electrodes into the front tip instead of the backplane of the body, as hereby a lower background resistance and a better stability of the signal could be retrieved.

The basolateral cavity of the FTPC (4) was modified by installation of a magnetic stirrer (5). The basolateral vessel, the connecting tubing and a piece of the peristaltic tubing were omitted. Thus the basolateral compartment volume was reduced from original 5.5 ml to 3.8 ml. Hydrostatic pressure balance was provided by means of adjustment of the height of the liquid column at the KRB buffer reservoir (8). Due to the design of the basolateral compartment the replenishment of the withdrawn sample volumes with blank KRB was provided automatically. For calculation of the permeated amount the sample volumes were taken into account.

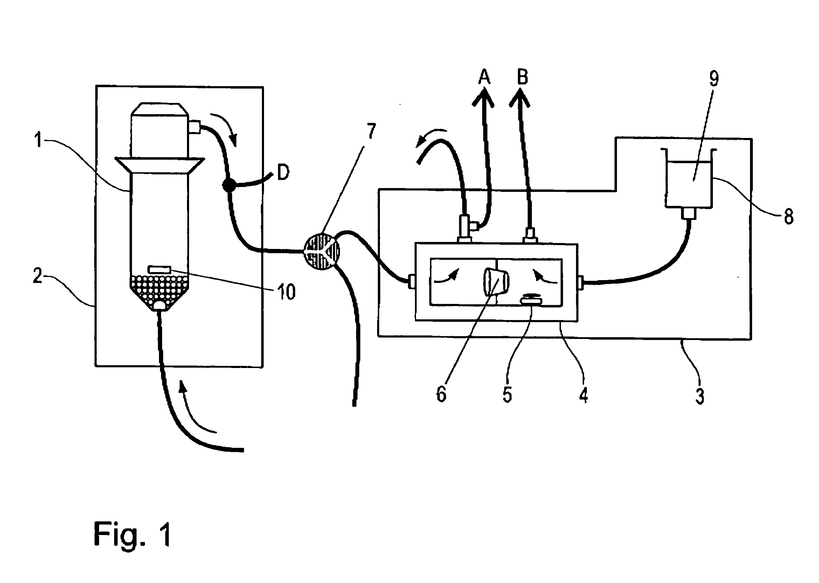

As shown in Figure 1 the dissolution module (2), an open compartment, consisted of a compendial flow through cell (1) that is described as apparatus four in the United States Pharmarcopeia (USP). The flow through dissolution cell (1) (e.g., Sotax CE1, Sotax, Lörrach, Germany) was linked with a flow through permeation cell (FTPC (4)) via a stream splitter (7). Samples of liquid, e.g., for concentration measurement of the sample of interest, were drawn automatically at sampling port D (Dissolution), in order to detect in vitro dissolution (release) profiles.

Permeation took place inside a specifically modified flow through permeation cell (FTPC (4)) (housing made of Polyetheretherketone (PEEK), Fig. 2). The FTPC (4) was divided in an apical and a basolateral compartment which were separated from each other by a Caco-2 cell monolayer grown on a 12 well Transwell® permeable support (Corning Incorporated, New York, NY, USA). Samples of liquid were taken automatically at sampling port A for the detection of the concentration of the sample of interest on the donor side of the cellular layer and at port B for the detection of the concentration of the sample of interest in the basolateral compartment after permeation across the cellular layer in order to determine the permeation profile.

The stream splitter (7) provided appropriate low flow rates for the permeation module (3) and divided the flow of the drug solution (obtained after dissolution of the drug substance or formulation) obtained by apparatus four USP (1) into a bigger waste stream and a smaller stream that was led into the apical compartment of the FTPC (4). Before the drug solution left the apical outflow of the FTPC (4), the molecules could permeate over the Caco-2 monolayer into the basolateral acceptor compartment.

As the basolateral side of the FTPC (4) was set up as a closed system, the volume of the samples drawn at port B had to be replenished with buffer solution (9). To warrant a homogeneous distribution inside the basolateral part of the FTPC (4), the acceptor compartment was equipped with a magnetic stirrer (5). During the experiment the dissolution cell and the FTPC (4) were immersed into a water bath and the temperature was adjusted to 37°C.

### SIA system

After modification of the EVOMX with a relay and installation of a multifunction USB-data acquisition device (NI-6009, National Instruments, Austin, TX) triggering of TEER measurement, A/D conversion of the signals and data collection could be performed by a PC using LabVIEW software (version 8.5.1, National Instruments, Austin, TX).

The SIA system consisted of a FIAIab 3500 (FIAIab instruments Inc, WA, USA) implemented with a 2.5 ml piston pump, a peristaltic pump and an eight port multiposition valve. Standards were aspirated by means of an autosampler (CetacASX 260, NE, USA).

SIA automation allowed sampling into 96 well plates. A second pump and valve module along with a second autosampler has been installed. By means of that a complete separation of the basolateral sampling route was established.

At sampling port D and A, a tubing was used to directly connect the stream containing the dissolved drug with the multiposition valve.

### Permeation of sodium fluorescein and cellular layer integrity

For quantification of sodium fluorescein a Cytofluor II fluorescence reader was used (λ_{exc} = 485 nm, λₑₘ = 530 nm) (PerSeptive Biosystems, Wiesbaden-Norderstedt, Germany).

The continuous TEER measurement was illustrated by use of Caco-2 cell monolayers and the dependency of their permeability for the paracellular transport marker sodium fluorescein on Ca²⁻ ions was recorded.

Culture of Caco-2 cells, and their use for the assay and Krebs Ringer Buffer (KRB) pH 7.4 were performed or made as described in sections 2.1. to 2.3 of Materials and methods of Motz et al. Eur J Pharm and Biopharm (2007) 66(2): 286-295. KRB was used as acceptor medium and as the basis for all types of donor solutions. The standard donor solution contained a concentration of 5 µg/ml sodium fluorescein in KRB. EDTA solutions containing 8 mM EDTA next to 5 µg/ml sodium fluorescein were prepared. Changes in osmolality that were caused by the addition of EDTA and the omission of the divalent salts were taken into account by means of adaption of the amount of NaCl to the calculative value of KRB. lsoosmolality of all solutions of 308 ± 4 mosmol/kg was checked via freezing point depression. Prior to each experiment, all buffer media were properly degassed by stirring under reduced air pressure.

After insertion of a transwell® into the FTPC (4) both compartments (apical and basolateral) were filled with KRB by switching on the apical pump and simultaneous application of the hydrostatic counter pressure upon the basolateral cavity. The flowrate through the apical compartment was 1.0 ml/min. After 10-15 min equilibration time the experiments were started by the activation of PC controlled TEER measurement and automated sampling. The first switch of donor solutions was conducted equally in all experiments from KRB to the standard donor solution, sodium fluorescein in KRB. This was done in order to proof the tightness of the cell monolayer. Afterwards the donor medium was switched to an EDTA solution for a certain time to get an effect on the monolayer and back again to the KRB solution containing sodium fluorescein.

The results of this experiment showed that the barrier properties of the Caco-2 monolayer inside the FTPC (4) could be maintained for at least 3 hours. Permeability of sodium fluorescein did not increase until TEER dropped below 300 Ω*cm². In all experiments blanc filter resistance values were subtracted. A single switch to 8 mM EDTA for 30 min and subsequent return to Ca²⁺ containing KRB led to a strong decrease of TEER to one third of the initial value.

## Claims

1. A method for measuring the permeability of a cellular layer for a sample of interest comprising the steps of
a) providing a flow-through permeation cell (FTPC (4)) comprising suitable electrodes (16, 17);
b) introducing a suitable carrier system for a cellular layer (6) into said FTPC (4);
c) filling the system with a suitable buffer solution at the apical and the basolateral side of said cellular layer;
d) introducing said sample of interest at the apical side of said cellular layer;
e) taking samples of liquid at the basolateral side or at the apical and the basolateral side of the cellular layer at certain time intervals;
f) determining the amount of said sample of interest in said samples of liquid taken and
g) determining the transepithelial electrical resistance (TEER) of the cellular layer in certain time intervals during the course of the permeation measurement
whereby the said electrodes (16, 17) are structurally integrated into the said FTPC (4).

2. The method according to claim 1 further comprising the step of
a) firstly providing a dissolution chamber, wherein the said sample of interest is placed for the determination of its dissolution properties;
b) taking samples of liquid at the outlet (D) of the dissolution chamber at certain time intervals during the course of the permeation measurement; and
c) determining the amount of the sample of interest in said samples of liquid taken.

3. The method according to claim 1 or 2, wherein adjustment of one or more assay parameters, test calibration, data acquisition and/or processing of obtained data is under the control of a control unit.

4. The method according to one or more of any preceding claims wherein the measurement of the TEER is performed in time intervals of 1 sec to 60 min.

5. The method according to one or more of any preceding claim, wherein said electrodes (16, 17) are at least partly structural integrated into the FTPC (4).

6. The method according to one or more of any preceding claim, wherein the cellular layer is of mesothelial or endothelial origin.

7. A device for measuring the transepithelial electrical resistance (TEER) of a cellular layer **characterized in that** it comprises
a) a flow-through permeation cell (FTPC (4)) designed to allow the introduction of a carrier system for the said cellular layer;
b) at least one pair of electrodes (16, 17) which are in contact with the apical and basolateral compartments of the said cellular layer, whereby
the said electrodes (16, 17) are structurally integrated into the said FTPC (4).

8. The device according to claim 7, wherein said electrodes (16, 17) are at least partly structural Integrated into the FTPC (4).

9. The device according to one or more of claims 7 to 8, wherein the said FTPC (4) has a liquid outlet at the at least basolateral side (B) of the cellular layer for sample taking

10. The device according to one or more of claims 7 to 9, which is under control of a control unit for the adjustment of assay parameters, device maintenance, test calibrations, data acquisition and/or processing of the obtained data.

11. A device for the measurement of permeation of a sample of interest through a cellular layer comprising
a) the device according to one or more of claims 7 to 10;
b) having a liquid outlet at the at least basolateral side (B) of the cellular layer for taking samples; and, optionally,
c) being under control of a control unit for the adjustment of assay parameters, device maintenance, test calibrations, data acquisition and/or processing of the obtained data.

12. A device for the measurement of dissolution of a sample of interest and the measurement of its permeation through a cellular layer comprising
a) the device as defined in claim 11; further comprising
b) a preceding dissolution chamber, wherein the said sample of interest is placed for the determination of its dissolution properties; and
c) having an opening for taking samples of liquid at the outlet of the dissolution chamber (D).

13. The use of electrodes (16, 17) for the at least partly structural integration into a flow-through permeation cell (FTPC (4)) designed to comprise a carrier system for a cellular layer (6) for the assessment of the transepithelial electrical resistance (TEER).

14. The use of the device according to one or more of claims 7 to 12 for the assessment of diffusion and/or permeation properties of a sample of interest through a cellular layer.
